(19) Europäisches Patentamt

European Patent Office

Office européen des brevets

(11) **EP 4 549 102 A1**

(12) **EUROPEAN PATENT APPLICATION**
published in accordance with Art. 153(4) EPC

(43) Date of publication:
**07.05.2025   Bulletin 2025/19**

(21) Application number: **23826225.7**

(22) Date of filing: **14.06.2023**

(51) International Patent Classification (IPC):
***B25J 9/16*** (2006.01)

(52) Cooperative Patent Classification (CPC):
**A61B 34/00; A61B 34/30; B25J 9/16;** Y02P 90/02

(86) International application number:
**PCT/CN2023/100137**

(87) International publication number:
**WO 2023/246576 (28.12.2023 Gazette 2023/52)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **22.06.2022   CN 202210711797**

(71) Applicant: **Ronovo (Shanghai) Medical Science
and
Technology Ltd.
Shanghai 201102 (CN)**

(72) Inventors:
• **XUE, Xiaojie**
  **Shanghai 201102 (CN)**
• **JIN, Xin**
  **Shanghai 201102 (CN)**
• **LI, Zhongqi**
  **Shanghai 201102 (CN)**

(74) Representative: **Murgitroyd & Company
165-169 Scotland Street
Glasgow G5 8PL (GB)**

(54) **ROBOTIC ARM ADJUSTMENT METHOD, APPARATUS, ELECTRONIC DEVICE AND STORAGE MEDIUM**

(57)    Disclosed in the present application are a robotic arm adjustment method, an apparatus, an electronic device and a storage medium. The method comprises: when it is determined that a robotic arm is located within or near an abnormal operating area, determining an adjustment direction for each redundant joint of a plurality of joints of the robotic arm; on the basis of a redundant joint position of each redundant joint at the current moment and the adjustment direction of each redundant joint, determining a first adjustment position of each redundant joint at a next moment; on the basis of each first adjustment position, determining a second adjustment position of each task joint of the plurality of joints except the redundant joints; and performing position adjustment on the robotic arm on the basis of the first adjustment position of each redundant joint and the second adjustment position of each task joint.

Determining an adjustment direction of each redundant joint of a plurality of joints of a robotic arm when it is determined that the robotic arm is located within or near an abnormal operating area — S110

↓

Determining a first adjustment position of each redundant joint at a next moment on the basis of a redundant joint position of each redundant joint at a current moment and the adjustment direction of each redundant joint — S120

↓

Determining a second adjustment position of each task joint of the plurality of joints except the redundant joints on the basis of each first adjustment position. — S130

↓

Performing position adjustment on the robotic arm on the basis of the first adjustment position of each redundant joint and the second adjustment position of each task joint. — S140

FIG. 1

EP 4 549 102 A1

## Description

The present application claims the right of the priority of Chinese patent application 202210711797.0 filed with China National Intellectual Property Administration on June 22, 2022, the contents of which are incorporated herein by reference in its entirety.

## TECHNICAL FIELD

The present disclosure relates to the technical field of surgical robot control, for example, to a robotic arm adjustment method, an apparatus, an electronic device, and a storage medium.

## BACKGROUND

In the process of minimally invasive surgery using a surgical robot, a robotic arm of the surgical robot continuously move during the surgery due to the surgical steps, making it easy for the robotic arm to reach a dangerous or hard-to-operate area, which affects the safety of the patient and the smooth progress of the surgery. Therefore, it is necessary to adjust the posture of the robotic arm away from the aforementioned special areas while ensuring that the surgery is performed without interruption, the trocar remains connected, and the instrument is in the current operating position, thus ensuring the normal progress of the surgery.

In related technologies, the following methods are used to adjust the robotic arm:

> 1. A method for moving the end effector of the robotic arm, such that the robotic arm is located in a safe area. The technique has the following technical problems during implementation: the surgery needs to be interrupted during adjustment; both the operator and the robotic arm need to be reset after adjustment.
> 2. An avoidance method for controlling the movement of the robotic arm based on the null space of the velocity Jacobian, such that the robotic arm is located in a safe area. The technique has the following technical problems during implementation: complex calculation process, large calculation errors, and poor anti-interference ability, thus reducing the safety and reliability of the surgery.

## CONTENT OF THE PRESENT INVENTION

The present disclosure provides a robotic arm adjustment method, an apparatus, an electronic device, and a storage medium, to realize the adjustment of a robotic arm without interrupting a surgery, thereby improving the safety and reliability of the adjustment of the robotic arm.

In a first aspect, the present disclosure provides a robotic arm adjustment method, comprising:

> determining an adjustment direction of each redundant joint of a plurality of joints of a robotic arm when it is determined that the robotic arm is located within or near an abnormal operating area;
> determining a first adjustment position of each redundant joint at a next moment on the basis of a redundant joint position of each redundant joint at a current moment and the adjustment direction of each redundant joint;
> determining a second adjustment position of each task joint of the plurality of joints except the redundant joints on the basis of each first adjustment position;
> performing position adjustment on the robotic arm on the basis of the first adjustment position of each redundant j oint and the second adjustment position of each task joint.

In a second aspect, the present disclosure also provides a robotic arm adjustment apparatus, comprising:

> an adjustment direction determination module, configured to determine an adjustment direction of each redundant joint of a plurality of joints of a robotic arm when it is determined that the robotic arm is located within or near an abnormal operating area;
> a first adjustment position determination module, configured to determine a first adjustment position of each redundant joint at a next moment on the basis of a redundant joint position of each redundant joint at a current moment and the adjustment direction of each redundant joint;
> a second adjustment position determination module, configured to determine a second adjustment position of each task joint of the plurality of joints except the redundant joints on the basis of each first adjustment position;
> a position adjustment module, configured to perform position adjustment on the robotic arm on the basis of the first adjustment position of each redundant joint and the second adjustment position of each task joint.

In a third aspect, the present disclosure also provides an electronic device, comprising:

> one or more processors;
> a storage device, configured to store one or more programs;
> when the one or more programs are executed by the one or more processors, the one or more processors are caused to implement the robotic arm adjustment method.

In a fourth aspect, the present disclosure also provides a computer-readable storage medium storing a computer program which, when executed by a processor, implements the robotic arm adjustment method.

BRIEF DESCRIPTION OF THE DRAWINGS

FIG. 1 is a schematic flow chart of a robotic arm adjustment method provided by an example of the present disclosure;
FIG. 2 is a schematic flow chart for determining an adjustment direction of a redundant joint provided by an example of the present disclosure;
FIG. 3A is a schematic structural diagram for adjusting a rectangular space of the robotic arm provided by an example of the present disclosure;
FIG. 3B is a schematic structural diagram of a rectangular coordinate adjustment coordinate system provided by an example of the present disclosure;
FIG. 3C is a schematic structural diagram for adjusting a corresponding joint space of the robotic arm provided by an example of the present disclosure;
FIG. 3D is a schematic structural diagram of a joint space adjustment coordinate system provided by an example of the present disclosure;
FIG. 4 is a schematic flow chart for determining a position conversion relationship provided by an example of the present disclosure;
FIG. 5 is a schematic flow chart of another robotic arm adjustment method provided by an example of the present disclosure;
FIG. 6 is a schematic structural diagram of a robotic arm adjustment apparatus provided by an example of the present disclosure;
FIG. 7 is a schematic structural diagram of an electronic device provided by an example of the present disclosure.

DETAILED DESCRIPTION OF THE PREFERRED EMBODIMENT

The present disclosure is described below in conjunction with drawings and examples. The specific examples described herein are merely intended to explain the present disclosure. For ease of description, only parts related to the present disclosure are illustrated in the drawings.

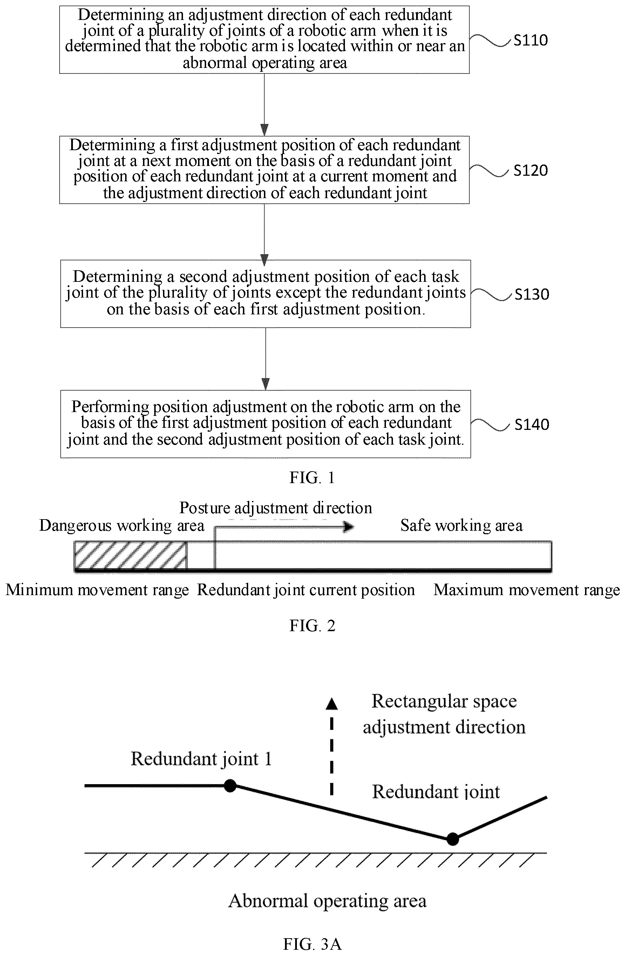

In some other examples, during the process of performing a surgery by a surgical robot, if a robotic arm of the surgical robot is located in an abnormal operating area for performing the surgery, the surgical risk is increased, leading to a decrease in the success rate of the operation. In order to enhance the safety of task operations, when the robotic arm of the surgical robot is detected to be located in an abnormal operating area for performing a surgery, it is necessary to accurately and quickly adjust the position of the robotic arm, such that the robotic arm is located in a normal operating area for performing the surgery. In order to ensure that the surgery can be performed smoothly during the position adjustment process, it is necessary to maintain the position of the trocar in the robotic arm of the surgical robot as well as the surgical instrument held by the end effector of the robotic arm to continue performing the surgery. In related technologies, the following methods are used to adjust the position of the robotic arm: 1. Moving the end effector of the robotic arm, such that the robotic arm is located in a safe area. However, the method needs to interrupt the surgery during adjustment, and after adjustment, both the operator and the robotic arm need to be reset. 2. Calculating the adjustment position based on the null space of the velocity Jacobian, and adjusting the robotic arm based on the adjustment position. However, the method involves a large amount of data calculation and is time-consuming, which cannot guarantee the safety of the surgery. Therefore, this example provides a robotic arm adjustment method, as shown in FIG. 1.

FIG. 1 is a schematic flow chart of a robotic arm adjustment method provided by an example of the present disclosure. This example may be applicable in the case of adjusting the position of a surgical robotic arm during the surgery. The method may be performed by a robotic arm adjustment apparatus, which may be implemented by means of software and/or hardware.

As shown in FIG. 1, the method includes the following steps:

S110, determining an adjustment direction of each redundant joint of a plurality of joints of a robotic arm when it is determined that the robotic arm is located within or near an abnormal operating area.
S120, determining a first adjustment position of each redundant joint at a next moment on the basis of a redundant joint position of each redundant joint at a current moment and the adjustment direction of each redundant joint.
S130, determining a second adjustment position of each task joint of the plurality of joints except the redundant joints on the basis of each first adjustment position.
S140, performing position adjustment on the robotic arm on the basis of the first adjustment position of each redundant joint and the second adjustment position of each task joint.

In an example of the present disclosure, the normal operating area can be understood as the predefined area where the surgical robot is performing a surgery, and accordingly, the abnormal operating area can be understood as the area other than the normal operating area. For example, during the execution of the surgery, the area where the robotic arm collides or is about to collide with the patient bed or other objects, the area where the robotic arm compresses or is about to compress the patient or nurse, and the area where the movement of the robotic arm is restricted can be understood as being located within or near the abnormal operating area.

Other areas that affect the execution of the surgery or affect the posture of the operation object during the execution of the surgery can also be understood as being located within or near the abnormal operating area. This example does not limit the location and range of areas located within or near the abnormal operating area.

In this example, during the process of performing a surgery by a surgical robot, it is determined whether a robotic arm of the surgical robot is located within or near the abnormal operating area, and based on the judgment result, it is determined whether the position of the robotic arm of the surgical robot needs to be adjusted.

The normal operating area of the robotic arm of the surgical robot is determined based on the surgical procedure performed by the surgical robot, and a robotic arm position threshold range of the robotic arm is determined based on a positional edge of the normal operating area. The position of the robotic arm is determined. If it is determined that the position of the robotic arm is not within the robotic arm position threshold range, the robotic arm is determined to be located in the abnormal operating area. If it is determined that the position of the robotic arm is within the robotic arm position threshold range and the distance from the boundary of the robotic arm position threshold range (*i.e.,* the positional edge of the normal operating area) is less than a preset first safety distance, the robotic arm is determined to be near the abnormal operating area. In this example, the method for determining the position of the robotic arm may include: obtaining the positions of a plurality of joints of the robotic arm and determining the position of the robotic arm of the surgical robot based on a preset position conversion relationship between the positions of the plurality of joints and the position of the robotic arm. Accordingly, the method for determining that the robotic arm is located within or near the abnormal operating area based on the position of the robotic arm at the current moment and the robotic arm position threshold may include: determining a joint position threshold range for each joint of the robotic arm based on the preset position conversion relationship and the robotic arm position threshold. The joint position of each joint at the current moment is determined, and the joint position of each joint is compared with the joint position threshold range. If any one of the joint positions is not within the joint position threshold range, it is determined that the robotic arm is located in the abnormal operating area. If any one of the joint positions is within the joint position threshold range and the distance from the boundary of the joint position threshold range is less than a preset second safety distance, it is determined that the robotic arm is near the abnormal operating area.

In an example, the preset position conversion relationship between the position of the robotic arm and the joint position is obtained, and the joint position threshold range for each joint of the robotic arm is determined based on the preset position conversion relationship and the robotic arm position threshold. The preset position conversion relationship may be an inverse kinematic expression, *i.e.,* the robotic arm position threshold is substituted as a parameter into the preset inverse kinematic expression to obtain the joint position threshold range for each joint of the robotic arm. The preset position conversion relationship may also be a pre-trained conversion model, *i.e.,* the robotic arm position threshold is input into the conversion model to obtain the joint position threshold range for each joint of the robotic arm output by the model. The joint position threshold range for each joint of the robotic arm can also be determined based on other forms of preset position conversion relationships, which is not limited in this example.

In an example, a plurality of joints are mounted with a positioning sensor configured to obtain a joint position of each joint based on a preset time interval, such that the joint position of each joint at each moment can be determined. The joint position of each joint is compared with the joint position threshold range. If any one of the joint positions is not within the joint position threshold range, it is determined that the robotic arm is located in the abnormal operating area.

The above method for determining whether the robotic arm is located in the abnormal operating area is only provided as an example. This example can also directly obtain the position of the robotic arm and directly determine whether the robotic arm is located in the abnormal operating area based on the position of the robotic arm. Additionally, the determination method can also be selected based on actual conditions, which is not limited in this example.

Based on the above example, when it is determined that the robotic arm is located within or near the abnormal operating area, the adjustment position of each joint is determined, thereby determining the adjustment position of the robotic arm. In this example, the joint of the robotic arm includes a redundant joint and a task joint. Specifically, the task joint is a joint necessary for performing the surgical procedure, while the redundant joint is a joint at the preset position prior to the surgery, which serves to maintain a preset angle between the robotic arms or between the robotic arm and other objects during the surgery, such as a preset angle between the robotic arm and the patient trolley.

In order to quickly determine the adjustment position of each joint of the robotic arm, the technical solution of this example first determines each redundant joint and each task joint of the robotic arm as well as a first adjustment position of the redundant joint at the next moment, and determines a second adjustment position of each task joint of the robotic arm on the basis of the first adjustment position of the redundant joint, so as to perform position adjustment on the robotic arm on the basis of the first adjustment position and the second adjustment position.

The method for determining the redundant joint of the plurality of joints may include: obtaining the number of constraints of the robotic arm during the surgery and the number of joints of the plurality of joints of the robotic arm; determining the number of redundant joints of the robotic arm based on the number of constraints and the number of joints; determining a degree of influence of each joint on the adjustment position at the next moment based on the preset position conversion relationship; determining each redundant joint of the plurality of joints based on the number of redundant joints and the degree of influence.

The number of constraints during the surgery is obtained and the number of the plurality of joints of the robotic arm is determined, and the number of redundant joints of the robotic arm is determined based on the number of constraints and the number of joints. For example, there are n joints in a robotic arm, and there are m constraints associated with the surgery. If n > m, the number of redundant joints of the robotic arm is calculated as n - m = r.

In this example, the degree of influence is used to characterize the risk of joint collision, and can be used to determine the joint that poses a high risk of collision to the robotic arm. The preset position conversion relationship between each joint and the robotic arm is determined based on the kinematic relationship of the robotic arm, and the risk degree of collision of each joint for the adjustment position of the robotic arm is determined based on the preset position conversion relationship.

Each redundant joint of the plurality of joints is determined based on the number of redundant joints and the degree of influence of each joint on the adjustment position. Exemplarily, the joint positions of the plurality of joints in the robotic arm are designated as position A, position B, and position C, and the robotic arm includes two redundant joints. Based on the kinematic relationship of the robotic arm, it is determined that the joints located at position A and position B in the robotic arm have the highest degree of influence on the adjustment position, and it is determined that the joints corresponding to position A and position B in the robotic arm are redundant joints.

In order to ensure that the surgical robot can continue performing the surgery without interruption during the position adjustment process, it is necessary to keep the position of the trocar connection of the surgical robot unchanged and to keep the posture and position of the end effector unchanged.

On the premise of satisfying the above requirements, the method for determining the adjustment direction of each redundant joint of the plurality of joints of the robotic arm in this example may include: For any one of the redundant joints at the current moment, based on the positional relationship between a current redundant joint and the abnormal operating area, determining the direction corresponding to the current redundant joint when moving the robotic arm away from the abnormal operating area as the adjustment direction of the current redundant joint.

The ranges of the normal operating area and the abnormal operating area are mapped to the adjustment range of the redundant joint. Within the adjustment range of the redundant joint, the direction corresponding to the current redundant joint when moving the robotic arm away from the abnormal operating area is determined as the adjustment direction of the current redundant joint.

Exemplarily, if the robotic arm includes only one redundant joint, referring to FIG. 2, the entire working area range (normal operating area) is mapped to the adjustment range of the redundant joint. Within the adjustment range of the redundant joint, at the current moment, the normal direction of the point on the edge of the nearest safe working area (normal operating area) away from the joint position is selected as the adjustment direction of the redundant joint at the next moment.

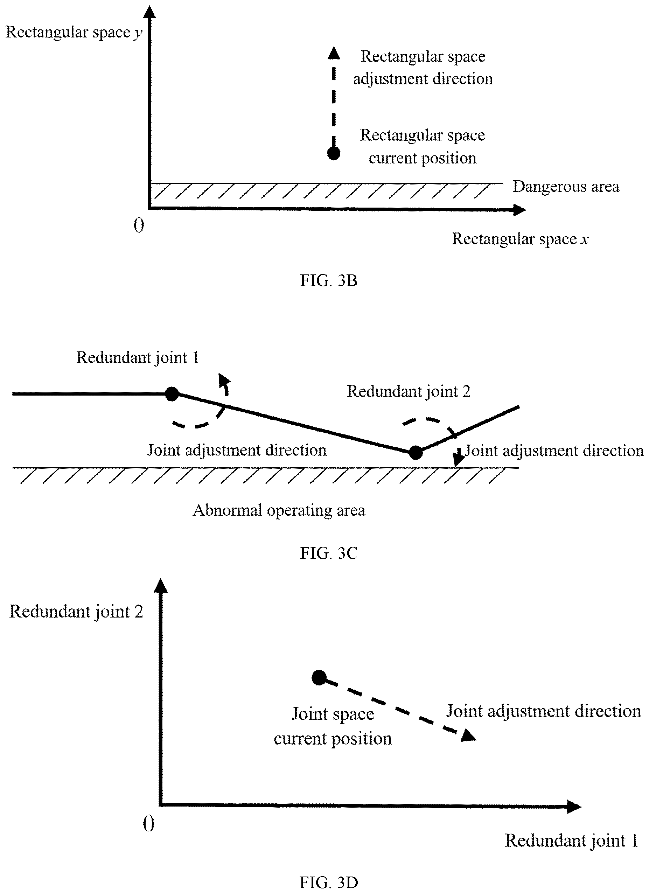

Exemplarily, if the robotic arm includes a plurality of redundant joints, this example is described taking example of including two redundant joints. Referring to FIG. 3A, the direction of the robotic arm away from the abnormal operating area between redundant joint 1 and redundant joint 2 is defined as an upward adjustment. Accordingly, when mapped to the Cartesian coordinate system, as shown in FIG. 3B, the adjustment direction corresponding to the redundant joint when the robotic arm is moved upwardly is the adjustment direction of the redundant joint at the next moment. Referring to FIG. 3C, during the process of determining the upward adjustment of the robotic arm, the adjustment direction of redundant joint 1 is an upward adjustment. In order to ensure that the posture and position of the end effector connected to redundant joint 2 remain unchanged, redundant joint 2 needs to be adjusted downward. Accordingly, when mapped to the Cartesian coordinate system, as shown in FIG. 3D, redundant joint 1 is adjusted upward while redundant joint 2 is adjusted downward.

Based on the above example, the first adjustment position of each redundant joint at the next moment is determined on the basis of the redundant joint position and adjustment direction of the redundant joint at the current moment.

For any one of the redundant joints, the method for determining the first adjustment position of the redundant joint at the next moment may include: setting a preset adjustment speed of the redundant joint, and determining the first adjustment position of the redundant joint at the next moment based on the redundant joint position, the adjustment direction, and the preset adjustment speed.

The preset adjustment speed of the redundant joint is set, and the first adjustment position of the redundant joint at the next moment is determined based on the joint position of the redundant joint at the current moment as well as the adjustment direction and the preset adjustment speed of the redundant joint. Exemplarily, the joint position of the redundant joint is denoted as $r = (r_1, r_2, ..., r_{n-m})^T$. The first adjustment position of the redundant joint at the next moment is determined based on the following expression:

$$r_i = r_{i-1} + v_{set} \cdot \Delta t$$

wherein $r_i$ represents the first adjustment position of the redundant joint at the next moment; $r_{i-1}$ represents the joint position of the redundant joint at the current moment; $v_{set}$ represents the preset adjustment speed set for the redundant j oint, wherein the preset adjustment speed includes the adjustment direction; $\Delta t$ represents the time interval between the current moment and the next moment

Based on the above example, the second adjustment position of each task joint of the plurality of joints except the redundant joints on the basis of each first adjustment position.

The method for determining the second adjustment position of each task joint of the plurality of joints may include: obtaining constraints for the robotic arm during the surgery; determining the second adjustment position corresponding to each task joint at the next moment based on the constraints and each first adjustment position.

In some examples, the robotic arm of the surgical robot includes two configurations. One configuration can be a separated design of the robotic arm, consisting of two sub-robotic arms with independent functions: an adjustment arm that is responsible for maintaining the position of the trocar during the surgery and a tool arm that executes task operations based on commands; wherein the tool arm is responsible for controlling the movement of instruments during the surgery, while the adjustment arm is responsible for adjusting the surgical position and the posture of the robotic arm. The other configuration can be an integrated design of the robotic arm, where the robotic arm simultaneously maintains the position of the trocar and executes task operations based on commands during the surgery. In order to realize the adjustment of the robotic arm away from the abnormal operating area during task operations, the entire robotic arm should include at least one redundant joint.

The robot trocar (*i.e.,* fixed point) is denoted as *RCM.* On the premise of keeping the position of the trocar connection unchanged, the adjustment constraint is to keep the position $P_{RCM}$ *(x, y, z)* of the trocar point after adjustment the same as the position $P_{RCMO}$(x, y, z) of the trocar point before adjustment, *i.e.,* the current adjustment constraint during the execution of the surgery, represented as $C_{RCM} = (c_{RCM1}, c_{RCM2}, c_{RCM3})^T$, satisfies the following condition:

$$\begin{cases} c_{RCM1} = P_{RCMx} - P_{RCMOx} = 0 \\ c_{RCM2} = P_{RCMy} - P_{RCMOy} = 0 \\ c_{RCM3} = P_{RCMz} - P_{RCMOz} = 0 \end{cases}$$

The robot trocar (*i.e.,* fixed point) is denoted as *TCP.* On the premise of keeping the posture and position of the end effector unchanged, the adjustment constraint is to keep the position $P_{TCP}$(x, y, z) and the posture $R_{TCP}$ (x, y, z) of the end effector after adjustment the same as the position $P_{TCPO}$(x, y, z) and the posture $R_{TCPO}$(x, y, z) of the end effector before adjustment, *i.e.,* the current adjustment constraint during the execution of the surgery, represented as $c_{TCP} = (c_{TCP1}, c_{TCP2}, ..., c_{TCP6})^T$, satisfies the following condition:

$$\begin{cases} c_{TCP1} = P_{TCPx} - P_{TCPOx} = 0 \\ c_{TCP2} = P_{TCPy} - P_{TCPOy} = 0 \\ c_{TCP3} = P_{TCPz} - P_{TCPOz} = 0 \\ c_{TCP4} = R_{TCPx} - R_{TCPOx} = 0 \\ c_{TCP5} = R_{TCPy} - R_{TCPOy} = 0 \\ c_{TCP6} = R_{TCPz} - R_{TCPOz} = 0 \end{cases}$$

Based on the above example, the condition for keeping the position of the trocar connection unchanged is collectively referred to as the *RCM* adjustment constraint, with the number of constraints $m_{RCM}$ = 3. The condition for keeping the posture and position of the end effector unchanged is referred to as the *TCP* adjustment constraint, with the number of constraints $m_{TCP}$ = 6.

For a separated designed robotic arm, the adjustment arm has a total of $n_t$ joints, and if it is necessary to keep the position of the trocar unchanged, then $n_t \geq m_{RCM}$; the tool arm has a total of $n_g$ joints, and if it is necessary to keep the position and posture of the end effector unchanged, then $n_g \geq m_{TCP}$. For an integrated designed robotic arm, all joints of the robotic arm should satisfy $n \geq m_{RCM} + m_{TCP}$.

Based on the above example, for an integrated designed robotic arm or a tool arm or adjustment arm that includes the redundant j oint, the second adjustment position corresponding to each task joint is determined based on the adjustment constraints and the first adjustment position of each redundant joint at the next moment.

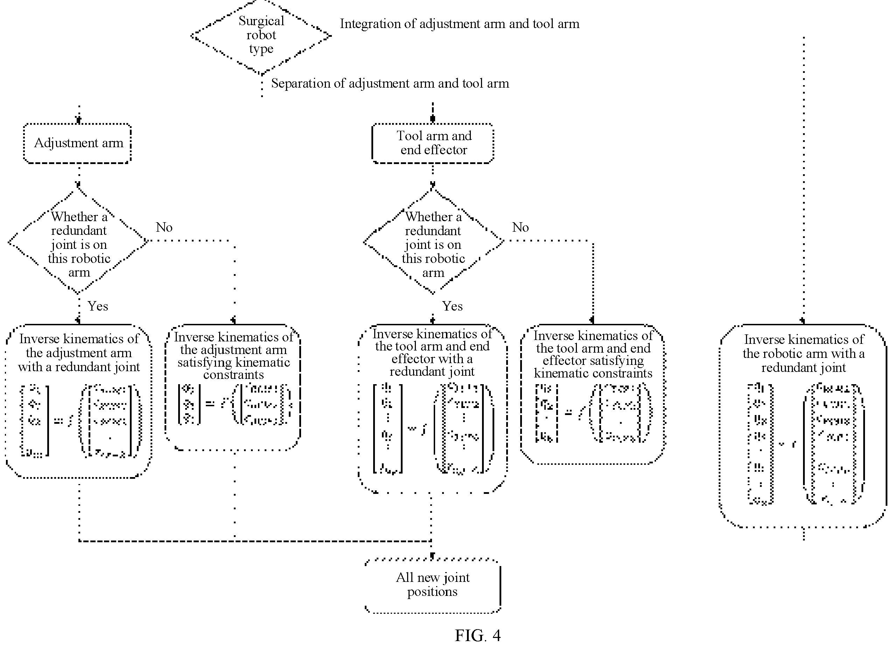

Referring to FIG. 4, the position conversion relationship between the second adjustment position of each task joint, the adjustment constraints, and the first adjustment position of each redundant joint at the next moment is constructed; wherein $q$ represents the second adjustment position of each task joint in the robotic arm; $c_{TCP}$ and $c_{RCM}$ represent the adjustment constraints; $r$ represents the first adjustment position of each redundant joint at the next moment; the function $f()$ represents the position conversion relationship between the second adjustment position of each task joint, the adjustment constraints, and the first adjustment position of each redundant joint at the next moment.

The method for solving the above position conversion relationship can involve geometric methods, coordinate transformation methods, or numerical iteration methods to determine the position conversion relationship between the second adjustment position of each task joint, the adjustment constraints, and the first adjustment position of each redundant joint at the next moment.

Additionally, a position conversion model between the second adjustment position of each task joint, the adjustment constraints, and the first adjustment position of each redundant joint at the next moment can be constructed through a neural network model, and the position conversion model can be trained to obtain a trained position conversion model.

Based on the solved position conversion relationship or the trained position conversion model, as well as the constraints and the first adjustment position of each redundant joint at the next moment, the second adjustment position of each task joint is determined. In the process of determining the adjustment position, analytical iterative operation or neural network model operation are used, which avoids numerical solution and high-dimensional matrix operation, thus improving computational efficiency and accuracy.

Based on the above example, position adjustment is performed on the robotic arm on the basis of the first adjustment position of each redundant joint and the second adjustment position of each task joint.

Based on the respective adjustment positions corresponding to the plurality of joints at the next moment, position adjustment is performed on the robotic arm to obtain the adjusted robotic arm.

The technical solution of this example involves determining an adjustment direction of each redundant joint of a plurality of joints of a robotic arm when it is determined that the robotic arm is located within or near an abnormal operating area; determining a first adjustment position of each redundant joint at the next moment on the basis of the redundant joint position and adjustment direction of the redundant joint; determining a second adjustment position of each task joint of the plurality of joints except the redundant joints on the basis of each first adjustment position; performing position adjustment on the robotic arm on the basis of the first adjustment position of each redundant joint and the second adjustment position of each task joint. It addresses the issues present in related technologies such as complex calculation process, large calculation errors, and poor anti-interference ability, realizing the adjustment of the robotic arm without interrupting the surgery, thereby improving the safety and reliability of the adjustment of the robotic arm.

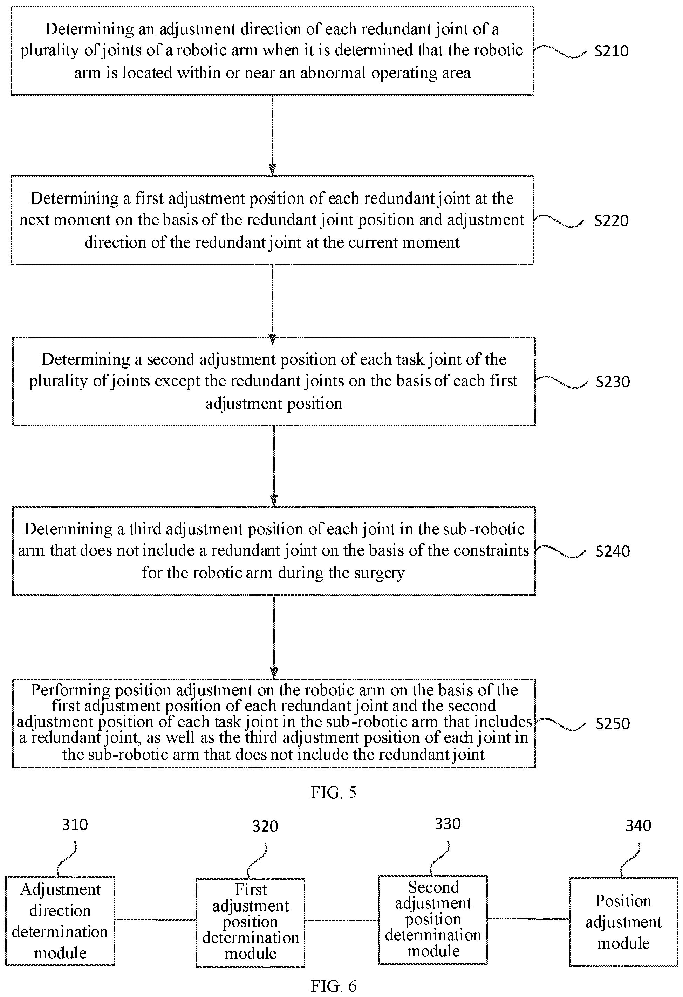

FIG. 5 is a flow chart of another robotic arm adjustment method provided by an example of the present disclosure. This example is implemented on the basis of any one of the technical solutions in the present disclosure, wherein the robotic arm includes two functionally independent sub-robotic arms. In the case where neither sub-robotic arm includes the redundant joint, the method further includes determining a third adjustment position of each joint in the sub-robotic arm that does not include the redundant joint based on the constraints for the robotic arm during the surgery. Accordingly, the robotic arm adjustment method includes performing position adjustment on the robotic arm based on the first adjustment position of each redundant joint and the second adjustment position of each task joint in a sub-robotic arm that includes a redundant joint, as well as the third adjustment position of each joint in the sub-robotic arm that does not include the redundant joint. The explanations of terms that are the same as or corresponding to those in the above examples are not repeated herein. Referring to FIG. 5, the robotic arm adjustment method provided by this example includes:

S210, determining an adjustment direction of each redundant joint of a plurality of joints of a robotic arm when it is determined that the robotic arm is located within or near an abnormal operating area.

S220, determining a first adjustment position of each redundant joint at the next moment on the basis of the redundant joint position and adjustment direction of the redundant joint at the current moment.

S230, determining a second adjustment position of each task joint of the plurality of joints except the redundant joints on the basis of each first adjustment position.

S240, determining a third adjustment position of each joint in the sub-robotic arm that does not include a redundant joint on the basis of the constraints for the robotic arm during the surgery.

S250, performing position adjustment on the robotic arm on the basis of the first adjustment position of each redundant joint and the second adjustment position of each task joint in the sub-robotic arm that includes a redundant joint, as well as the third adjustment position of each joint in the sub-robotic arm that does not include the redundant joint.

In an example of the present disclosure, for a separated designed robotic arm, when the redundant joint is present only in either of the sub-robotic arms, i.e., only in the adjustment arm or the tool arm, the method for determining a third adjustment position of each joint in the sub-robotic arm that does not include the redundant joint includes: determining a third adjustment position of each joint in the sub-robotic arm that does not include the redundant joint based on the constraints for the robotic arm during the surgery.

Continuing with reference to FIG. 4, the position conversion relationship between the third adjustment position of each joint at the next moment and the adjustment constraints is constructed; wherein $q$ represents the third adjustment position of each joint in the robotic arm at the next moment; $c_{TCP}$ and $c_{RCM}$ represent the adjustment constraints; the function $f()$ represents the position conversion relationship between the third adjustment position of each joint at the next moment and the adjustment constraints.

The method for solving the above position conversion relationship can involve geometric methods, coordinate transformation methods, or numerical iteration methods to determine the position conversion relationship between the third adjustment position of each joint at the next moment and the adjustment constraints. Additionally, a position conversion model between the third adjustment position of each joint at the next moment and the adjustment constraints can be constructed through a neural network model, and the position conversion model can be trained to obtain a trained position conversion model.

Based on the solved position conversion relationship or the trained position conversion model and the constraints, the third adjustment position of each joint at the next moment is determined. In the process of determining the adjustment position, analytical iterative operation or neural network model operation are used, which avoids numerical solution and high-dimensional matrix operation, thus improving computational efficiency and accuracy.

Based on the above example, position adjustment is performed on the robotic arm on the basis of the first adjustment position of each redundant joint and the second adjustment position of each task joint in the sub-robotic arm that includes the redundant joint, as well as the third adjustment position of each joint in the sub-robotic arm that does not include the redundant joint.

Based on the respective adjustment positions corresponding to the plurality of joints at the next moment, position adjustment is performed on the robotic arm to obtain the adjusted robotic arm.

The technical solution of this example involves determining an adjustment direction of each redundant joint of a plurality of joints of a robotic arm when it is determined that the robotic arm is located within or near an abnormal operating area; determining a first adjustment position of each redundant joint at the next moment on the basis of the redundant joint position and adjustment direction of the redundant joint; determining a second adjustment position of each task joint of the plurality of joints except the redundant joints on the basis of each first adjustment position; determining a third adjustment position of each joint in the sub-robotic arm that does not include the redundant joint based on the constraints for the robotic arm during the surgery; performing position adjustment on the robotic arm on the basis of the first adjustment position of each redundant joint and the second adjustment position of each task joint in the sub-robotic arm that includes the redundant joint, as well as the third adjustment position of each joint in the sub-robotic arm that does not include the redundant joint. It realizes the adjustment of a plurality of robotic arms without interrupting the surgery, thereby improving the reliability of the adjustment of the robotic arm.

The following is an example of a robotic arm adjustment apparatus provided by an example of the present disclosure, which belongs to the same concept as the robotic arm adjustment method in the above example. For details that are not elaborated in the example of the robotic arm adjustment apparatus, please refer to the above example of the robotic arm adjustment method.

FIG. 6 is a schematic structural diagram of a robotic arm adjustment apparatus provided by an example of the present disclosure. Referring to FIG. 6, the robotic arm adjustment apparatus includes the following structures: an adjustment direction determination module 310, a first adjustment position determination module 320, a second adjustment position determination module 330, and a position adjustment module 340; wherein the adjustment direction determination module 310 is configured to determine an adjustment direction of each redundant joint of a plurality of joints of a robotic arm when it is determined that the robotic arm is located within or near an abnormal operating area; the first adjustment position determination module 320 is configured to determine a first adjustment position of each redundant joint at a next moment on the basis of a redundant joint position of each redundant joint at a current moment and the adjustment direction of each redundant joint; the second adjustment position determination module 330 is configured to determine a second adjustment position of each task joint of the plurality of joints except the redundant joints on the basis of each first adjustment position; the position adjustment module 340 is configured to perform position adjustment on the robotic arm on the basis of the first adjustment position of each redundant joint and the second adjustment position of each task joint.

The technical solution of this example involves determining an adjustment direction of each redundant joint of a plurality of joints of a robotic arm when it is determined that the robotic arm is located within or near an abnormal operating area; determining a first adjustment position of each redundant joint at a next moment on the basis of a redundant joint position of each redundant joint at a current moment and the adjustment direction of each redundant joint; determining a second adjustment position of each task joint of the plurality of joints except the redundant joints on the basis of each first adjustment position; performing position adjustment on the robotic arm on the basis of the first adjustment position of each redundant joint and the second adjustment position of each task joint. It addresses the issues present in related technologies such as complex calculation process, large calculation errors, and poor anti-interference ability, realizing the adjustment of the robotic arm without interrupting the surgery, thereby improving the safety and reliability of the adjustment of the robotic arm.

Based on any one of the above technical solutions, the apparatus further includes:

A joint position threshold determination unit, configured to obtain a robotic arm position threshold for the normal operating area of the robotic arm before determining the adjustment direction of each redundant joint of the plurality of joints of the robotic arm, and determine a joint position threshold range for each joint of the robotic arm based on the preset position conversion relationship and the robotic arm position threshold; a position comparison unit, configured to determine a joint position of each joint at the current moment and compare the joint position with the joint position threshold range for each joint; an abnormal operating area determination unit, configured to determine that the robotic arm is located in the abnormal operating area if any one of the joint positions is not within the joint position threshold range for the joint.

Based on any one of the above technical solutions, the adjustment direction determination module 310 includes:

A constraint quantity and joint quantity acquisition unit, configured to obtain the number of constraints of the robotic arm during the surgery and the number of joints of the plurality of joints of the robotic arm; a redundant joint quantity determination unit, configured to determine the number of redundant joints of the robotic arm based on the number of constraints and the number of joints; a degree of influence determination unit, configured to determine a degree of influence of each joint on the adjustment position based on the preset position conversion relationship; a redundant joint determination unit, configured to determine each redundant joint of the plurality of joints based on the number of redundant joints and the degree of influence.

Based on any one of the above technical solutions, the adjustment direction determination module 310 includes:

An adjustment direction determination unit, configured to, for any one of the redundant joints at the current moment, based on the positional relationship between a current redundant joint and the abnormal operating area, determine the direction corresponding to the current redundant joint when moving the robotic arm away from the abnormal operating area as the adjustment direction of the current redundant joint.

Based on any one of the above technical solutions, the first adjustment position determination module 320 includes:

A first adjustment position determination unit, configured to obtain a preset adjustment speed of the redundant joint, and determine a first adjustment position of the redundant joint at the next moment based on the redundant joint position, the adjustment direction, and the preset adjustment speed.

Based on any one of the above technical solutions, the second adjustment position determination module 330 includes:

An adjustment constraint acquisition unit, configured to obtain constraints for the robotic arm during the surgery; a second adjustment position determination unit, configured to determine a second adjustment position corresponding to each task joint at the next moment based on the adjustment constraints and each first adjustment position.

Based on any one of the above technical solutions, the robotic arm includes two functionally independent sub-robotic arms. In the case where neither sub-robotic arm includes the redundant joint, the apparatus further includes:

A third adjustment position determination module, configured to determine a third adjustment position of each joint in the sub-robotic arm that does not include the redundant joint on the basis of the constraints for the robotic arm during the surgery. Accordingly, the position adjustment module 340 includes:

A position adjustment unit, configured to perform position adjustment on the robotic arm on the basis of the first adjustment position of each redundant joint and the second adjustment position of each task joint in the sub-robotic arm that includes the redundant joint, as well as the third adjustment position of each joint in the sub-robotic arm that does not include the redundant joint.

The robotic arm adjustment apparatus provided by an example of the present disclosure can execute the robotic arm adjustment method provided by any example of the present disclosure, and has functional modules and effects corresponding to the execution method.

In the above example of the robotic arm adjustment apparatus, the multiple units and modules included are only divided according to functional logic, but are not limited to the above divisions, as long as they can achieve the corresponding functions; in addition, the names of the multiple functional units are merely for ease of distinction and are not used to limit the scope of protection of the present disclosure.

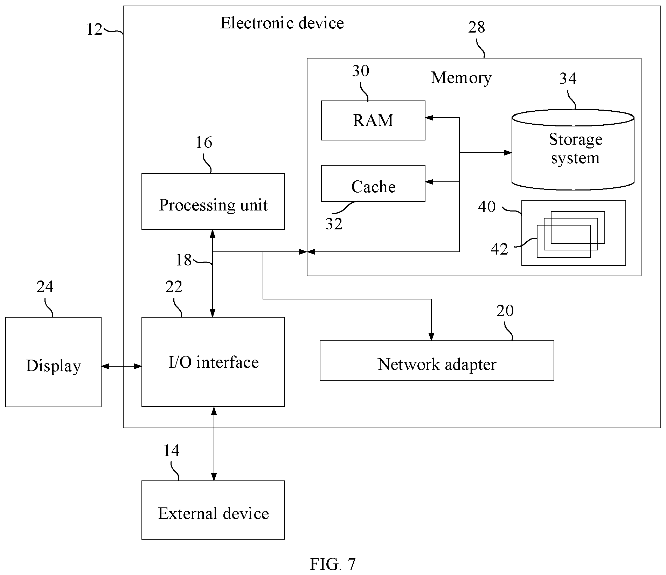

FIG. 7 is a schematic structural diagram of an electronic device provided by an example of the present disclosure. FIG. 7 shows a block diagram of an exemplary electronic device 12 suitable for implementing an embodiment of the present disclosure. The electronic device 12 shown in FIG. 7 is only an example and should not impose any limitations on the functions and scope of use of the examples of the present disclosure.

As shown in FIG. 7, the electronic device 12 is represented in the form of a general computing electronic device. The components of the electronic device 12 may include, but are not limited to: one or more processors or processing units 16, a memory 28, and a bus 18 connecting different components (including the memory 28 and the processing unit 16).

The bus 18 represents one or more of several types of bus structures, including a memory bus or a memory controller, a peripheral bus, a graphics accelerated port, a processor, or a local bus using any of a variety of bus structures. For example, these architectures include, but are not limited to, the Industry Standard Architecture (ISA) bus, the Micro Channel Architecture (MCA) bus, the enhanced ISA bus, the Video Electronics Standards Association (VESA) local bus, and the Peripheral Component Interconnect (PCI) bus.

The electronic device 12 includes a variety of computer system readable media. These media may be any available media that can be accessed by the electronic device 12, including volatile and non-volatile media, as well as removable and non-removable media.

The memory 28 may include a computer system readable medium in the form of volatile memory, such as random access memory (RAM) 30 and/or cache 32. The electronic device 12 may include other removable/non-removable and volatile/non-volatile computer system storage media. By way of example only, a storage system 34 may be configured to read and write to a non-removable, non-volatile magnetic medium (not shown in FIG. 7, commonly referred to as a "hard disk drive"). Although not shown in FIG. 7, a disk drive configured to read and write to a removable non-volatile disk (*e.g.,* "floppy disk"), and a optical disc drive configured to read and write to a removable non-volatile optical disk (*e.g.,* compact disc read-only memory (CD-ROM), digital video disc-read only memory (DVD-ROM), or other optical media) may be provided. In these cases, each drive may be connected to the bus 18 via one or more data medium interfaces. The memory 28 may include at least one program product having a set (*e.g.,* at least one) of program modules that are configured to perform the functions of the examples of the present disclosure.

A program/utility 40 having a set (at least one) of program modules 42 may be stored in, for example, the memory 28. These program modules 42 include, but are not limited to, an operating system, one or more application programs, other program modules, and program data. Each of these examples or a combination thereof may include an implementation of a network environment. The program modules 42 generally perform the functions and/or methods in the examples described in the present disclosure.

The electronic device 12 may also communicate with one or more external devices 14 (*e.g.,* keyboard, pointing device, and display 24), and may also communicate with one or more devices that enable a user to interact with the electronic device 12, and/or with any device (*e.g.,* network card and modem) that enables the electronic device 12 to communicate with one or more other computing devices. Such communication may take place via an input/output (I/O) interface 22. Furthermore, the electronic device 12 may also communicate with one or more networks (*e.g.,* local area network (LAN), wide area network (WAN), and/or a public network, such as the Internet) via a network adapter 20. As shown in FIG. 7, the network adapter 20 communicates with other modules of the electronic device 12 via the bus 18. It should be understood that, although not shown in FIG. 7, other hardware and/or software modules may be used in conjunction with the electronic device 12, including, but not limited to: microcode, device drivers, redundant processing units, external disk drive arrays, redundant array of independent disks (RAID) systems, tape drives, data backup storage systems, *etc.*

The processing unit 16 executes a variety of functional applications and sample data acquisition by running programs stored in the memory 28, for example, implementing the steps of a robotic arm adjustment method provided by the examples of the present disclosure. The robotic arm adjustment method comprises:
determining an adjustment direction of each redundant joint of a plurality of joints of a robotic arm when it is determined that the robotic arm is located within or near an abnormal operating area; determining a first adjustment position of each redundant joint at a next moment on the basis of a redundant joint position of each redundant joint at a current moment and the adjustment direction of each redundant joint; determining a second adjustment position of each task joint of the plurality of joints except the redundant joints on the basis of each first adjustment position; performing position adjustment on the robotic arm on the basis of the first adjustment position of each redundant joint and the second adjustment position of each task joint.

It can be understood by those skilled in the art that the processor can also implement the technical solution of the robotic arm adjustment method provided by any example of the present disclosure.

This example also provides a computer-readable storage medium storing a computer program which, when executed by a processor, for example, implements the steps of a robotic arm adjustment method provided by the examples of the present disclosure. The robotic arm adjustment method comprises:
determining an adjustment direction of each redundant joint of a plurality of joints of a robotic arm when it is determined that the robotic arm is located within or near an abnormal operating area; determining a first adjustment position of each redundant joint at a next moment on the basis of a redundant joint position of each redundant joint at a current moment and the adjustment direction of each redundant joint; determining a second adjustment position of each task joint of the plurality of joints except the redundant joints on the basis of each first adjustment position; performing position adjustment on the robotic arm on the basis of the first adjustment position of each redundant joint and the second adjustment position of each task joint.

The computer storage medium in the example of the present disclosure may be any combination of one or more computer-readable media. The computer-readable medium may be a computer-readable signal medium or a computer-readable storage medium. The computer-readable storage medium may include, for example, but is not limited to: an electrical, magnetic, optical, electromagnetic, infrared, or semiconductor-based system, apparatus, or device, or any combination of the above. Examples (a non-exhaustive list) of the computer-readable storage medium include: electrical connections having one or more conductors, portable computer disks, hard drives, RAM, ROM, erasable programmable read-only memory (EPROM or flash memory), optical fibers, CD-ROM, optical storage devices, magnetic storage devices, or any suitable combination of the above. In this document, the computer-readable storage medium may be any tangible medium that contains or stores a program for use by or in conjunction with an instruction execution system, apparatus, or device.

The computer-readable signal medium may include a data signal propagated in a baseband or as part of a carrier wave carrying a computer-readable program code therein. Such propagated data signals may take a variety of forms, including, but not limited to, electromagnetic signals, optical signals, or any suitable combination of the above. The computer-readable signal medium may also be any computer-readable medium other than the computer-readable storage medium that can send, propagate, or transmit a program for use by or in conjunction with an instruction execution system, apparatus, or device.

The program code embodied on the computer-readable medium may be transmitted using any appropriate medium, including, but not limited to, wireless, wire, optical cable, radio frequency (RF), *etc.,* or any suitable combination of the above.

Computer program code for executing the operations of the present disclosure may be written in one or more programming languages or combinations thereof. The programming languages include object-oriented programming languages such as Java, Smalltalk, and C++, as well as conventional procedural programming languages such as "C" or similar programming language. The program code may be executed entirely on the user's computer, partly on the user's computer, as a stand-alone software package, partly on the user's computer and partly on a remote computer, or entirely on a remote computer or server. In situations involving remote computers, the remote computer may be connected to the user's computer through any kind of network, including a LAN or WAN, or it may be connected to an external computer (*e.g.,* using an Internet service provider to connect through the Internet).

Those of ordinary skill in the art should understand that the plurality of modules or steps of the present disclosure may be implemented using general computing devices. These modules or steps may be centralized on a single computing device or distributed across a network consisting of a plurality of computing devices. Optionally, these modules or steps may be implemented with a program code executable by a computing device, such that they may be stored in a storage device for execution by the computing device, or these modules or steps may be fabricated into a plurality of integrated circuit modules, or fabricated into a single integrated circuit module. Thus, the present disclosure is not limited to any specific combination of hardware and software.

## Claims

1. A robotic arm adjustment method, comprising:

   determining an adjustment direction of each redundant joint of a plurality of joints of a robotic arm when it is determined that the robotic arm is located within or near an abnormal operating area;

   determining a first adjustment position of each redundant joint at a next moment on the basis of a redundant joint position of each redundant joint at a current moment and the adjustment direction of each redundant joint;

   determining a second adjustment position of each task joint of the plurality of joints except the redundant joints on the basis of each first adjustment position;

   performing position adjustment on the robotic arm on the basis of the first adjustment position of each redundant joint and the second adjustment position of each task joint.

2. The method according to claim 1, wherein a method for determining that the robotic arm is located within the abnormal operating area comprises:

   obtaining a robotic arm position threshold for a normal operating area of the robotic arm, and determining a joint position threshold range for each joint of the robotic arm based on a preset position conversion relationship and the robotic arm position threshold;

   determining a joint position of each joint at a current moment, and comparing the joint position with the joint position threshold range for each joint;

   determining that the robotic arm is located within the abnormal operating area in the case where a certain joint position is not within the joint position threshold range for a certain joint.

3. The method according to claim 1, before determining an adjustment direction of each redundant joint of a plurality of joints of the robotic arm, further comprising:

   obtaining the number of constraints of the robotic arm during a surgery and the number of joints of the plurality of joints of the robotic arm;

   determining the number of redundant joints of the robotic arm based on the number of constraints and the number of joints;

   determining a degree of influence of each joint on the adjustment position based on a preset position conversion relationship;

   determining each redundant joint of the plurality of joints based on the number of redundant joints and the degree of influence.

4. The method according to claim 3, wherein determining an adjustment direction of each redundant joint of a plurality of joints of the robotic arm comprises: determining, for a certain redundant joint at a current moment, a direction corresponding to a current redundant joint when moving the robotic arm away from the abnormal operating area as an adjustment direction of the current redundant joint based on a positional relationship between the current redundant joint and the abnormal operating area.

5. The method according to claim 1, wherein determining a first adjustment position of each redundant joint at a next moment on the basis of a redundant joint position of each redundant joint at a current moment and the adjustment direction of each redundant joint comprises:

obtaining a preset adjustment speed of the redundant joint, and determining a first adjustment position of the redundant joint at a next moment based on the redundant joint position, the adjustment direction, and the preset adjustment speed.

6. The method according to claim 1, wherein determining a second adjustment position of each task joint of the plurality of joints except the redundant joints on the basis of each first adjustment position comprises:

obtaining constraints for the robotic arm during a surgery;
determining a second adjustment position corresponding to each task joint based on the constraints and each first adjustment position.

7. The method according to claim 1, wherein the robotic arm comprises two functionally independent sub-robotic arms;
in the case where a sub-robotic arm does not comprise the redundant joint, the method further comprises:

determining a third adjustment position of each joint in the sub-robotic arm that does not comprise the redundant joint on the basis of constraints for the robotic arm during a surgery;
performing position adjustment on the robotic arm on the basis of the first adjustment position of each redundant joint and the second adjustment position of each task joint comprises:
performing position adjustment on the robotic arm on the basis of the first adjustment position of each redundant joint and the second adjustment position of each task joint in a sub-robotic arm that comprises the redundant joint, as well as the third adjustment position of each joint in the sub-robotic arm that does not comprise the redundant joint.

8. A robotic arm adjustment apparatus, comprising:

an adjustment direction determination module, configured to determine an adjustment direction of each redundant joint of a plurality of joints of a robotic arm when it is determined that the robotic arm is located within or near an abnormal operating area;
a first adjustment position determination module, configured to determine a first adjustment position of each redundant joint at a next moment on the basis of a redundant joint position of each redundant joint at a current moment and the adjustment direction of each redundant joint;
a second adjustment position determination module, configured to determine a second adjustment position of each task joint of the plurality of joints except the redundant joints on the basis of each first adjustment position;
a position adjustment module, configured to perform position adjustment on the robotic arm on the basis of the first adjustment position of each redundant joint and the second adjustment position of each task joint.

9. An electronic device, comprising:

at least one processor;
a storage device, configured to store at least one program;
when the at least one program is executed by the at least one processor, the at least one processor is caused to implement the robotic arm adjustment method according to any one of claims 1 to 7.

10. A computer-readable storage medium storing a computer program which, when executed by a processor, implements the robotic arm adjustment method according to any one of claims 1 to 7.

Determining an adjustment direction of each redundant joint of a plurality of joints of a robotic arm when it is determined that the robotic arm is located within or near an abnormal operating area    ⌐⌐S110

Determining a first adjustment position of each redundant joint at a next moment on the basis of a redundant joint position of each redundant joint at a current moment and the adjustment direction of each redundant joint    ⌐⌐S120

Determining a second adjustment position of each task joint of the plurality of joints except the redundant joints on the basis of each first adjustment position.    ⌐⌐S130

Performing position adjustment on the robotic arm on the basis of the first adjustment position of each redundant joint and the second adjustment position of each task joint.    ⌐⌐S140

FIG. 1

Posture adjustment direction

Dangerous working area ⌐————————→ Safe working area

Minimum movement range    Redundant joint current position    Maximum movement range

FIG. 2

▲ Rectangular space
⫶ adjustment direction
⫶
Redundant joint 1    ⫶
⫶
⫶ Redundant joint

Abnormal operating area

FIG. 3A

Rectangular space $y$

Rectangular space
adjustment direction

Rectangular space
current position

Dangerous area

0

Rectangular space $x$

FIG. 3B

Redundant joint 1

Redundant joint 2

Joint adjustment direction

Joint adjustment direction

Abnormal operating area

FIG. 3C

Redundant joint 2

Joint space
current position

Joint adjustment direction

0

Redundant joint 1

FIG. 3D

FIG. 4

Determining an adjustment direction of each redundant joint of a plurality of joints of a robotic arm when it is determined that the robotic arm is located within or near an abnormal operating area — S210

Determining a first adjustment position of each redundant joint at the next moment on the basis of the redundant joint position and adjustment direction of the redundant joint at the current moment — S220

Determining a second adjustment position of each task joint of the plurality of joints except the redundant joints on the basis of each first adjustment position — S230

Determining a third adjustment position of each joint in the sub-robotic arm that does not include a redundant joint on the basis of the constraints for the robotic arm during the surgery — S240

Performing position adjustment on the robotic arm on the basis of the first adjustment position of each redundant joint and the second adjustment position of each task joint in the sub-robotic arm that includes a redundant joint, as well as the third adjustment position of each joint in the sub-robotic arm that does not include the redundant joint — S250

FIG. 5

310
Adjustment direction determination module

320
First adjustment position determination module

330
Second adjustment position determination module

340
Position adjustment module

FIG. 6

FIG. 7

## INTERNATIONAL SEARCH REPORT

| International application No. |
|---|
| **PCT/CN2023/100137** |

### A. CLASSIFICATION OF SUBJECT MATTER

B25J9/16(2006.01)i

According to International Patent Classification (IPC) or to both national classification and IPC

### B. FIELDS SEARCHED

Minimum documentation searched (classification system followed by classification symbols)

IPC: B25J

Documentation searched other than minimum documentation to the extent that such documents are included in the fields searched

Electronic data base consulted during the international search (name of data base and, where practicable, search terms used)

CNTXT, ENTXTC, VEN, CNKI: 机械臂, 机器人, 冗余, 关节, 碰撞, 避障, 障碍, 调整, 调节, 位置, mechanical, arm?, robot?, redundant, joint?, obstacle?, collision, adjust+, position

### C. DOCUMENTS CONSIDERED TO BE RELEVANT

| Category* | Citation of document, with indication, where appropriate, of the relevant passages | Relevant to claim No. |
|---|---|---|
| X | CN 113119096 A (UBTECH ROBOTICS CORP.) 16 July 2021 (2021-07-16) description, paragraphs 48-103, and figures 1-4 | 1-10 |
| A | US 5150026 A (N.A.S.A.) 22 September 1992 (1992-09-22) entire document | 1-10 |
| A | CN 103009389 A (BEIJING INSTITUTE OF CONTROL ENGINEERING) 03 April 2013 (2013-04-03) entire document | 1-10 |
| A | CN 106625666 A (GUANGZHOU SHIYUAN ELECTRONIC TECHNOLOGY CO., LTD.) 10 May 2017 (2017-05-10) entire document | 1-10 |
| A | CN 112605996 A (SUN YAT-SEN UNIVERSITY) 06 April 2021 (2021-04-06) entire document | 1-10 |
| A | US 2015127151 A1 (KUKA LAB. G.M.B.H.) 07 May 2015 (2015-05-07) entire document | 1-10 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **31 August 2023** | **12 September 2023** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/100137**

**C. DOCUMENTS CONSIDERED TO BE RELEVANT**

| Category* | Citation of document, with indication, where appropriate, of the relevant passages | Relevant to claim No. |
|---|---|---|
| A | US 5737500 A (CALIFORNIA INSTITUTE OF TECHNOLOGY) 07 April 1998 (1998-04-07)<br>    entire document | 1-10 |

Form PCT/ISA/210 (second sheet) (July 2022)

## INTERNATIONAL SEARCH REPORT
### Information on patent family members

| International application No. |
| --- |
| **PCT/CN2023/100137** |

| Patent document cited in search report | | | Publication date (day/month/year) | Patent family member(s) | | | Publication date (day/month/year) |
| --- | --- | --- | --- | --- | --- | --- | --- |
| CN | 113119096 | A | 16 July 2021 | US | 2021387340 | A1 | 16 December 2021 |
| US | 5150026 | A | 22 September 1992 | None | | | |
| CN | 103009389 | A | 03 April 2013 | None | | | |
| CN | 106625666 | A | 10 May 2017 | WO | 2018107851 | A1 | 21 June 2018 |
| CN | 112605996 | A | 06 April 2021 | None | | | |
| US | 2015127151 | A1 | 07 May 2015 | KR | 20150051892 | A | 13 May 2015 |
| | | | | EP | 2868445 | A1 | 06 May 2015 |
| | | | | DE | 102013222456 | A1 | 07 May 2015 |
| | | | | CN | 104608127 | A | 13 May 2015 |
| US | 5737500 | A | 07 April 1998 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210711797